(19) **Europäisches Patentamt**
**European Patent Office**
**Office européen des brevets**

(11) **EP 1 187 550 B1**

(12) **EUROPEAN PATENT SPECIFICATION**

(45) Date of publication and mention
of the grant of the patent:
**17.06.2009  Bulletin 2009/25**

(51) Int Cl.:
***A61B 5/00*** *(2006.01)*    ***A61B 5/12*** *(2006.01)*

(21) Application number: **00926442.5**

(86) International application number:
**PCT/US2000/011389**

(22) Date of filing: **28.04.2000**

(87) International publication number:
**WO 2000/065983 (09.11.2000 Gazette 2000/45)**

(54) **HANDHELD AUDIOMETRIC DEVICE AND METHOD OF TESTING HEARING**

HANDGEHALTENES AUDIOMETRISCHES GERÄT UND VERFAHREN FÜR HÖRTEST

DISPOSITIF AUDIOMETRIQUE A MAIN ET PROCEDE POUR REALISER UN TEST AUDITIF

(84) Designated Contracting States:
**AT BE CH CY DE DK ES FI FR GB GR IE IT LI LU
MC NL PT SE**

(30) Priority: **29.04.1999  US 131542 P**

(43) Date of publication of application:
**20.03.2002  Bulletin 2002/12**

(73) Proprietors:
• **Causevic, Elvir**
**Fenton, MO 63026 (US)**
• **Causevic, Eldar**
**Fenton, MO 63026-2813 (US)**

(72) Inventors:
• **Causevic, Elvir**
**Fenton, MO 63026 (US)**

• **Causevic, Eldar**
**Fenton, MO 63026-2813 (US)**

(74) Representative: **Dennemeyer, John James et al
Dennemeyer & Associates S.A.
Patents-trademarks-Designs
P.O. Box 1502
1015 Luxembourg (LU)**

(56) References cited:
**WO-A-98/43566          DE-A- 4 234 782
US-A- 5 197 332          US-A- 5 267 571
US-A- 5 601 091          US-A- 5 738 633
US-A- 5 868 682          US-A- 5 885 225**

EP 1 187 550 B1

## Description

<u>Technical Field</u>

This invention relates to the field of auditory measurement devices and associated screening methods. In particular, the invention relates to a hand-held auditory measurement device, which has features beneficial to all neonatal screening programs. While the invention is described with particular emphasis to its auditory screening application, those skilled in the art will recognize the wider applicability of the inventive principles disclosed hereinafter.

<u>Background Art</u>

Universal neonatal auditory screening programs have expanded greatly because of improved auditory measurement capability, improved rehabilitation strategies, increased awareness of the dramatic benefits of early intervention for hearing impaired babies, and changes in governmental policies. Current neonatal auditory screening approaches, however, do not account adequately for the many different types and degrees of auditory abnormalities that are encountered with present screening approaches. Because of this, individual screening tests based on a single measurement can be influenced negatively by interaction among various independent auditory abnormalities. Current screening approaches have not considered adequately the entire screening program including (i) physical characteristics of the measurement device i.e., portability, physical size and ease of use, (ii) operational characteristics of the device i.e., battery life, amount of record storage, required operating training, etc. and/or (iii) program logistics i.e., retesting mechanisms, referral mechanisms, record processing, patient tracking, report writing, and other practical aspects. These factors can interact negatively to increase the total cost of an auditory screening program, including the primary economic cost of screening, testing, the secondary economic cost of additional testing, and non-economic costs such as parental anxiety incurred when provided with incorrect information.

These costs, both actual and human, can be reduced by reducing the cost per test, reducing the false positive rate, and resolving false positive screening results at the bedside prior to hospital discharge. The cost per screening can be reduced with a dedicated device optimized for screening in any location and enhanced to allow effective operation by minimally trained personnel. The performance characteristic of the device of our invention includes reduced measurement time, the ability to operate and configure without an external computer, the ability to integrate and interpret all test results, the ability to store large number of test results, long battery life, and bi-directional wireless transfer of data to and from external devices.

We have found false positive results can be reduced in two ways. First, the initial screening test performance can be improved with enhanced signal processing, more efficient test parameters, and by combining different types of tests. Second, false positive rates also can be reduced by providing a mechanism for resolving an initial screening test failure at the bedside at the time of the initial screening. This capability is provided through the availability of an automated screening auditory brainstem response (ABR) test capability provided by the same device. Secondly, operational processes of a screening program can be improved through the use of several onboard computer based expert systems. These computer based expert systems provide improved automatic interpretation of single test results, automatic interpretation of multiple test results, and improved referral processes through the matching of local referral sources with various test outcomes, such as a referral to a specific type of follow-up, whether it be a pediatrician, audiologist, otolaryngologist, or a nurse. The device disclosed hereinafter integrates in a single, hand-held device, a single stimulus transducer, a single processor and a single software application for otoacoustic emission (OAE), ABR testing, tympanometry and otoreflectance, as well as OAE simulator.

An auditory abnormality is not a single, clearly defined entity with a single cause, a single referral source and a single intervention strategy. The peripheral auditory system has three separate divisions, the external ear, the middle ear, and the sensorineural portion consisting of the inner ear or cochlea. and the eight cranial nerve. Abnormalities can and do exist independently in all three divisions and these individual abnormalities require different intervention and treatment. Prior art physical and operational characteristics of devices and their influences on program logistics can interact negatively to increase the total cost of an auditory screening program. The primary economic cost is the cost of each screening test though this is not the only economic cost. A screening test failure is called a "refer" and usually is resolved with an expensive full diagnostic test scheduled several weeks after hospital discharge, resulting in significant economic cost. A substantial portion of these costs is unnecessary if the screening false positive rate is high. Non economic costs include parental anxiety for false positive screening results, unfavorable professional perception of program effectiveness for programs with high false positive rates and even inappropriate professional intervention because of misleading screening results.

The intervention of multiple measurements into a single hand-held instrument allows for very important new functionality not available with existing neonatal auditory screening devices. This functionality includes (1) detection of common external and middle ear abnormalities; (2) the detection of less common sensorineural hearing loss associated with outer hair cell abnormalities, and (3) the detection of even less common sensorineural hearing loss associated with inner hair cell or auditory nerve abnormality. Moreover, the device disclosed hereinafter has the potential to improve the accuracy and reliability of OAE measurements, to allow for optimal interpretation of both the OAE and ABR results, and to improve the referral process.

Attempts have been made in the past to provide the capabilities provided by the present invention. In particular, U.S. Patent Nos. 5,601,091 ('091) and 5,916,174 ('174) disclose audio screening apparatus which purport to provide a hand-held portable screening device. However, the screening device disclosed in those patents is used in conjunction with a conventional computer, and requires a docking station for full applicational use. In no way does the disclosure of either patent provide a hand-held device that can be used independently of any other computer. That is to say, the invention disclosed hereinafter provides a device of significantly reduced size i.e., hand-held, which is capable of providing OAE and ABR testing, as well as tympanometry otoreflectance, and OAE simulator. It can be operated in a stand-alone mode, independently of any other computer connection, if desired. The device includes a patient database, with names, and full graphic display capability. The device also preferably is provided with a wireless infrared and an RS 232 connection port to provide output directly to printers or to a larger database where such is required.

The '174 and '091 patents also operate on a linear averaging method to remove background noise. While such method works well for its intended purposes, use of a linear averaging method is time consuming. Consequently, we developed a frame overlap method for rejecting noise and improving signal reliability in a device which measures, in the embodiment illustrated, 7 ¼" x 3 ¾" x 1 ½".

US 5,885,225 discloses an auditory screening device as well a method of conducting an auditory test according the preambles of claim 1 and claim 25 respectively.

Summary of Invention

The invention is defined by the appended claims. One of the objects of this invention is to provide a reduced size hand-held device for auditory screening which provides OAE, ABR, tympanometry, otoreflectance and OAE simulator operation.

Another object of this invention is to provide an audio screening device, which is hand-held and operates in a fully stand-alone mode, operating independently of any other computer connection.

Another object of this invention is to provide a hand-held device that provides a patient database on the device.

Another objection of this invention is to provide a hand-held audio screening apparatus that provides for full graphic display on the device itself.

Another object of this invention is to provide a device that increases noise rejection and reduces processing time through the use of frame overlapping techniques.

A further object of this invention is to provide a device with ABR testing that automates electrode impedance checking prior to test.

Another object of this invention is to provide a device which is low in cost, and which can be adapted to provide a wide ranging of auditory screening applications.

In accordance with this invention, generally stated, an effective auditory screening method and device according to claim 25 and claim 1, respectively are provided. The integration of an OAE screening device and ABR screening device into a single, hand-held instrument enables a user to detect less common sensorineural hearing loss associated with outer hair cell abnormalities and the detection of less common sensor hearing loss associated with inner hair cell abnormalities. In the preferred embodiment, the device includes a portable hand-held enclosure containing a digital signal processor. The processor has a computer program associated with it, capable of conducting both otoacoustic emission test procedures and auditory brainstem response test procedures for a test subject. A display device is mounted to the enclosure, and displays patient information, test setup procedure, and test results including graphing of test results. The enclosure includes a connection point for a probe, the connection point being operatively connected to the signal processor. The device also includes an onboard power supply, making the device completely self contained.

A method of testing OAE response in a test subject is provided which utilize a unique method of noise reduction to provide acceptable data even in high level ambient noise conditions of the test subject's environment.

Brief Description Of The Drawings

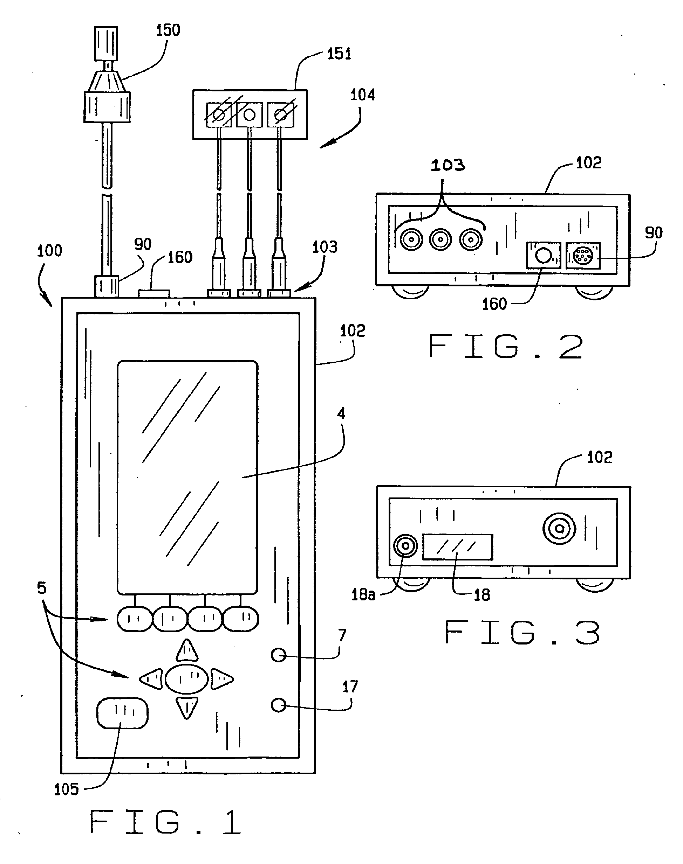

In the drawings, Figure 1 is a top plan view of one illustrative embodiment of audio screen device of the present invention.

Figure 2 is a view in end elevation;
Figure 3 is a view in end elevation of the end opposite to that shown in Figure 2
Figure 4 is a block diagrammatic view of the device shown in Figure 1;
Figure 5 and 6 are block diagrammatic views of the algorithm employed with the device of Figure 1 in connection with ABR testing;
Figure 7 is a diagrammatic view of frame sliding implemented by the algorithm of Figure 4; and
Figure 8 is a block diagrammatic view of the algorithm implemented with respect to OAE testing to improve the signal to noise ratio employed with the device of Figure 1.

Best Mode for Carrying out the Invention

The following detailed description illustrates the invention by way of example and not by way of limitation. This description will clearly enable one skilled in the art to make and use the invention, and describes several embodiments, adaptations, variations, alternatives and uses of the invention, including what we presently believe is the best mode for carrying out the invention. It will nevertheless be understood that no limitation in the scope of the invention is thereby intended, and that alterations and further modifications of the illustrative devices is contemplated, including but not limited to further applications of the principles of the invention illustrated herein as would normally occur to one skilled in the art to which this invention relates.

Referring now to Figures 1 - 3, reference numeral 100 illustrates one embodiment of the audio screening device of the present invention. The screening device 100 includes an enclosure 102, which in the preferred embodiment, and for purposes of illustration and not for limitation, measures 7 ¼" long by 3 ¾" wide by 1 ½" deep. It is important to note that the device 100 can be carried by the user without compromise, and truly represents a portable hand-held device having full functionality as described below. The device 100 includes a keyboard 5, an LCD display 4, an LED pass/refer indicator 7, and an LED AC charging indicator 17. Again, by way of illustration and not by limitation, it should be noted that the screen 4 measures, in the preferred embodiment, approximately 2" by 3 3/8". The measurement is not necessarily important, except to show that the LCD display is fully functional for a user, and the unit can operate independently of any other computer system. In the embodiment illustrated, the enclosure 102 also houses an infrared port 18, a compatible RS-232 port 18a, a probe connection 90 for an ear probe 150, and an interface 103 for a plurality of electrodes 104. The electrodes 104 are shown attached to a conventional carrier 151.

Ear probe 150 is conventional and is not described in detail. Suitable probes are commercially available from Etymotic Research, Part No. ER-10C, for example.

A novel feature of this invention is the provision of an OAE simulator ear probe interface 160. The simulator function permits a user to test the integrity of the entire OAE test system, by providing active feedback and simulation of a test subject's ear.

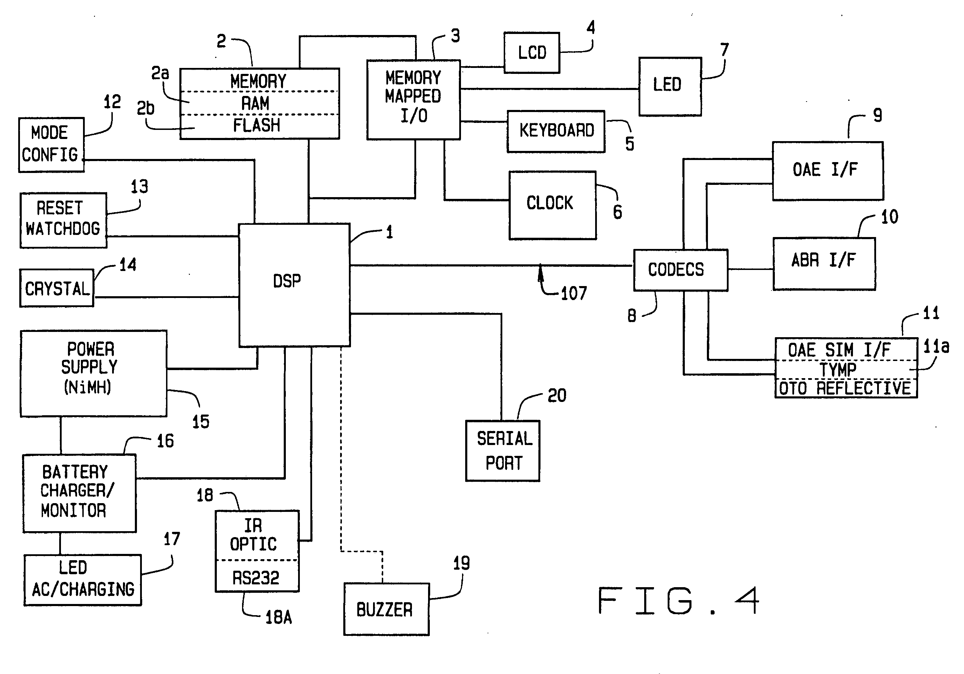

Referring now to Figure 4, a block diagram view of the device 100 is shown and described. The device 100 contains OAE, ABR and OAE simulator capabilities in a single, hand-held package. Preferably, the system shown in Figure 4 is manufactured on a single printed circuit board, with mixed signal design for both analog and digital operation. The device preferably is low powered, and generally operates at 3.3 volts, except for the LCD 4 and some low power portions of the analog circuitry employed with the device 100.

A digital signal processor 1 is the control for the device 100. In the preferred embodiment illustrated, the processor 1 is a Motorola chip DSP 56303. All signal processing functions described hereinafter are performed by the processor 1, as well as the control of all input and output functions of the device 100. In addition, the graphic functions, user interface, patient data storage functions and other device functionality are controlled by the processor 1. In conventional design logic, the digital signal processor 1 is used for signal processing, and a separate micro controller is used for device control. We have been able to eliminate the separate microprocessor, resulting in substantial savings in space, cost and power consumption.

A memory subsystem 2 is operatively connected to the processor 1. The memory subsystem 2 includes a random access memory 2a for storing intermediate results and holding temporary variables, and a flash memory 2b for storing non-volatile, electrically programmable variables, patient data and configuration information. In the embodiment illustrated, the flash memory 2b is substantially oversized, enable the device 100 to accommodate as many as 300 full patient records, as well as multiple configurations files.

A memory mapped input/output device 3 is operatively connected to the memory subsystem 2 and to the digital signal processor 1. The memory mapped input/output 3 in turn is operatively connected to the LCD display 4, the keyboard 5, the pass/referral LED indicator 7 and a real time clock 6.

The LCD display 4 is the largest non-custom LCD available. While custom LCD displays can be obtained, they add prohibitive cost to the product. The LCD display 4 provides the user with 128 x 256 pixels of graphics. That display is sufficient to present full waveforms of audiometric tests conducted by the device 100. The keyboard 5 preferably is a membrane switch keyboard, which incorporates only the minimum keys necessary for operation of the device 100. All keys are programmable, except for the on/off key 105.

A real time clock 6 is operatively connected to the processor 1 through the memory mapped device 3. The clock 6 enables the processor 1 to provide a time stamp for each patient and test performed, as well as providing time signals for internal operation of the device 100.

The LED pass/refer diode 7 is used to convey test results to non-trained users, namely a nurse as opposed to an audiologist or otolaryngologist. Use of the LED 7 avoids confusion or misinterpretation of the LCD graphics display 4, and allows use of the device 100 in low light areas, where the LCD display 4 may be difficult to interpret.

The plurality of analog to digital/digital to analog coder/decoders 8 (codecs 8) is operatively connected to the signal processor 1. As will be appreciated by those skilled in the art, the codecs 8 are special integrated circuit chips that perform analog to digital and digital to analog conversion. The codecs 8 are operatively connected to the signal processor 1 along a dedicated serial link indicated by the reference numeral 107. The codecs 8 in turn are operatively associated with a plurality of input/ output devices, which provide the functionality of the device 100 under control of the processor 1.

An otoacoustic emission interface 9 is operatively connected to the signal processor 1 through the associated codecs 8. The interface 9 preferably is a low noise, differential analog circuit with high common mode noise rejection. The interface 9 is intended to drive two sound transducers inserted in the ear canal which produce a variety of signals, from pure tones at various frequencies to chirps, clicks, sinc waveforms etc. The otoacoustic emission interface 9 can present tones at all standard audiometric frequencies and sound pressure levels. The device employed with the interface 9 includes a microphone, also inserted in the ear canal, which collects signals coming back from the ear, and provides sufficient linear amplification to present the signals to the codecs 8. In various embodiments of this invention, the interface 9 also can be used for otoreflectance measurements for assessing some middle ear conditions.

The ABR interface 10 consists of a plurality of analog signal processing chips, not shown individually, which filter and amplify the signals connected from the scalp of a subject via electrode wires 104. In this mode of operation, the ear is presented with a repeated auditory stimulus, which causes firing of the eighth nerve, and the associated nerve pass into the brainstem. As those firings occur, electrical potentials are generated all the way to the scalp, and there they are detected by the electrodes 104. An additional function of the interface 10 is to provide automated impedance check of the placement of electrodes. Once the electrodes are in place, a small current is injected through the electrodes into the scalp of the subject, and the impedance between electrodes is measured. Impedance can be varied by placement of the electrodes. Once the impedance is within the predetermined range for operation, ABR signal connection can begin. It is important to note that impedance checking can be accomplished without unplugging the electrodes. That is to say checking is automatic. As latter described in greater detail, the measured ABR response is based on the detection of a peak in the waveform in a point approximately up to 15 milliseconds after a sound click, depending upon gestational age or patient age. The actual latency of this peak is then compared to the latency of this peak in normal hearing neonates or adults.

The otoacoustic emission simulator interface 11 is used to check the integrity of the OAE system. It includes a transducer or speaker and a microphone. The microphone collects the signals presented by the OAE probe, presents them to the codecs 8 and processor 1 for signal processing, and then the speaker presents the corresponding tone at the correct frequency and amplitude back to the original OAE probe thus providing an active, calibrated test cavity.

Our invention optionally may include a tympanometry interface 11a in place of the interface 11. The tympanometry interface 11a comprises an electronic output channel to drive a miniature pump, not shown, which can produce pressure or a vacuum in the ear canal of a test subject. A corresponding pressure sensor is used to measure this pressure, and the signal from the pressure sensor is fed into an analog input of the codecs 8. The signal can be used as an independent feature, and the device will show full graphics output on the LCD 4 in real time. In the alternative, this test may be used in combination with the OAE or ABR test to compensate for middle ear conditions.

A mode configuration system 12, a reset watchdog system 13, a crystal clock 14, a power supply 15 and a battery charger 16 all are also positioned within the enclosure 102 and operatively connected to the processor 1. While each of these blocks is required for operation of the device 102, they are standard in nature and are not described in detail.

The processor 1 has an input output channel 18, which are preferably an infrared connection and an isolated RS-232 interface. The device 100 can communicate with any infrared compatible or RS-232 compatible personal computer, printer, or other digital device for data transmission. Data transmission may include patient information, configuration data for the signal processor 1, or software program updates.

A buzzer 19 also is provided. The buzzer 19 provides an audio feedback to the user for keyboard actions and audio indication for error conditions.

A serial port 20 also is operative connected to the processor 1. The serial port 20 is utilized to provide direct programming of the processor 1 from a personal computer, for example, and is intended for use only for initial software download and major software program upgrades of the processor 1.

A distortion product otoacoustic emission (DPOAE) is a tone generated by a normal ear in response to the application of two external tones. When two tones, $f_1$ and $f_2$ are applied to an ear, the normal non-linear outer hair cells generate a third tone $f_{dp}$, which is called a distortion product. $F_{dp}$ then propagates from the outer hair cells back to the ear canal where it is emitted. The level of the DPOAE can be used as a measure of outer hair cell function. If the outer hair cell system is absent or otherwise not functioning properly, the non-linearity will be absent or reduced and the $f_{dp}$ will either not be generated or generated at a lower than expected level.

The measured DPOAE is highly dependent upon the specific tones that invoke it. The frequencies of $f_1$ and $f_2$, and their respective levels in the ear canal, L1 and L2 must be controlled precisely. Under known signal conditions, the largest distortion product is generated at a very specific frequency ($f_{dp} = 2 f_1 - f_2$), and level $L_{dp.}$ Comparison of the level of $L_{dp}$ with known values from individuals with normal outer hair cell systems forms the basis of the decision of whether the patient either passed the screening (pass/refer LED 7) or requires a referral for a more complete diagnostic testing.

Signals other than pure tones can be presented to the ear, which will also evoke a response from the ear, such as clicks, chirps, etc. DPOAE is used to as an example, the other stimuli would be processed the same way.

The processor 1 utilizes a unique method of detecting signals for the OAE test. While the method is a time domain sum and average operation, the key concept is to reuse data from adjacent frames to average with the current frame. This method is described for the purpose of this specification as "sliding". The limit to the size of the overlap is the auto correlation of original data. The method works on the assumption that the data within the overlap frames is different, and that the noise is uncorrelated. It is key to keep the frame size an integer number (one or more) of the original data cycles.

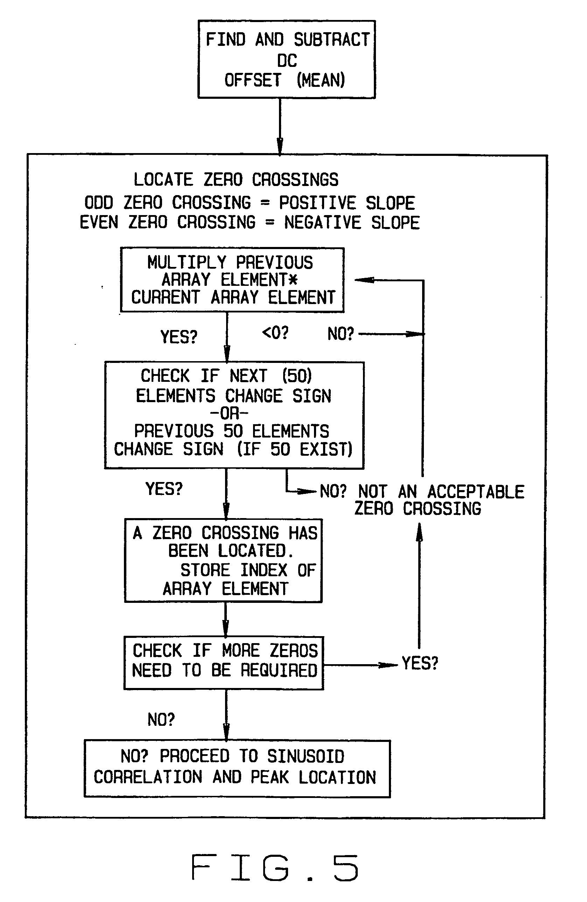

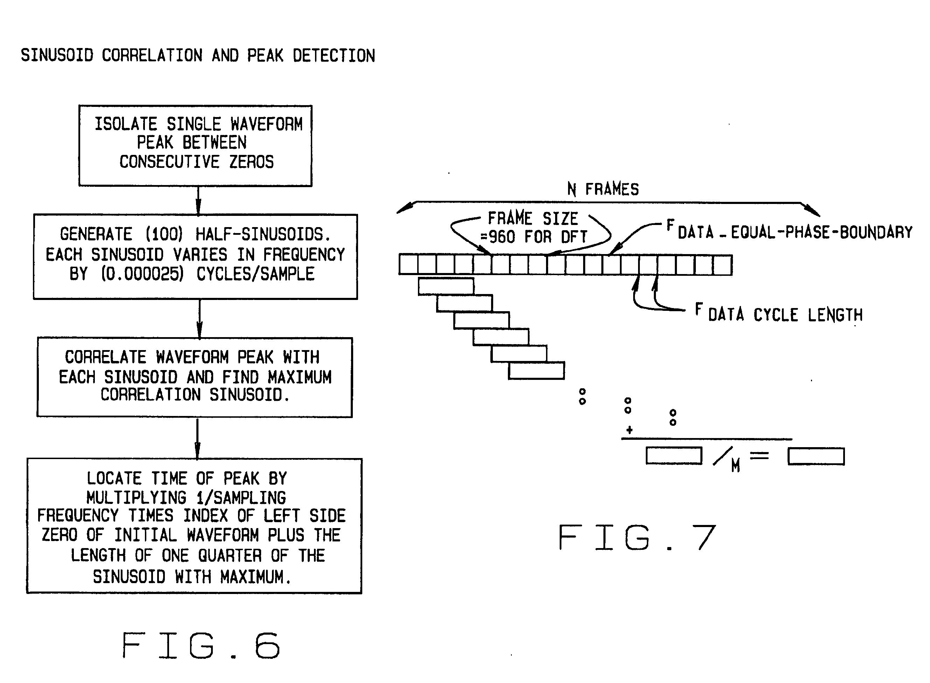

The important difference between the method of the present invention and linear averaging is that the overlapping number M (sum operation) equals ((frame number divided by (frame size minus 1)) times (frames size divided by (frame data cycle length plus 1))) which is larger than the received data frame number by a factor by which the previous frame is slid. Therefore, the expected performance of this method is better than standard linear averaging. In this method, the frame size divided by frame data cycle length must be an integer. The method is shown diagrammatically in Figure 5 and Figure 6.

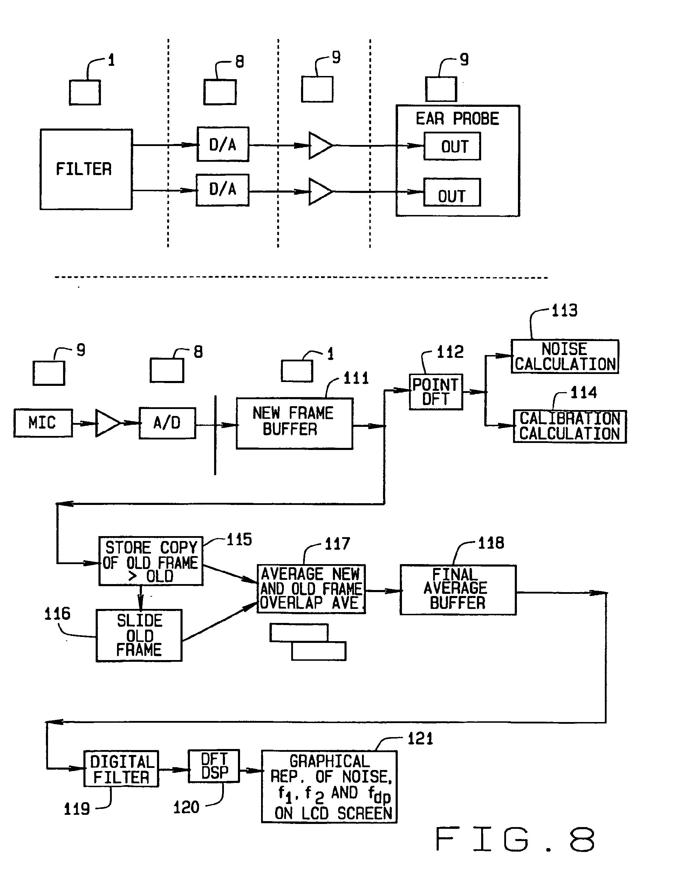

The processor 1 algorithm is implemented and explained with reference to Figure 7 and Figure 8. As there shown, the processor 1 sends an output through the digital analog converter portion of the codecs 8 through the OAE interface 9 to the ear probe utilized in conjunction with the device 100. The ear probe includes a microphone which returns signals through the interface 9 and the codecs 8 to a new frame buffer 111 in the processor 1. The size of the new frame buffer 111 is calculated to be an integer number of samples of the two primary tones at frequencies f1 and f2, and also, an integer number of samples of the otoacoustic tone produced by the ear at $f_{dp}$. This is a critical step to assure quality of subsequent signaling processing, by avoiding windowing techniques, which can introduce substantial artifacts. Tables of numbers for each standard frequency employed in the device 100 and for other frequencies in use or intended for use in the device 100 are available, and are programmed into the algorithm once the user selects the test frequencies. Should a combination of frequencies be required for which no common integer number can be found to fit in a practical size frame, the frame size is adjusted to $f_{dp}$ and the frame is windowed prior to Fourier Transformation, but this method is used only in extreme cases since in normal operation, the required frequencies are available.

The data from the single frame is passed to a point Discrete Fourier Transform 112 (DFT) block which calculates the signal's magnitude and phase content, but only at frequencies of interest, including $f_1$, $f_2$, $f_{dp}$ to determine a noise floor. Windowing is induced prior to DFT to reduce edge effects, although windowing induces energy at other bands. The block 112 is used only for temporary calculations, and the windowed data is not reused again. The output of block 112 is the magnitude and phase of primary signals at $f_1$ and $f_2$ and the noise floor figure of time at $f_{dp}$. The output of block 112 forms an input to frame rejection block 113 and to an on-line calibration calculation block 114.

With the information on the magnitudes at various frequencies, a noise calculation algorithm is employed at and around $f_{dp}$ to determine the noise floor. The magnitude of the noise floor and frequency content are used against a set of predetermined conditions i.e. a comparison against an empirically derived table contained in the processor 1, to determine the outcome of the frame. That outcome has three distinct possibilities. First, if the noise magnitude and frame content exceed a multi-threshold condition at measured frequency bands, the new frame is rejected. Second, if the noise magnitudes fall between a set of reject thresholds and a set of accept thresholds, the data in the frame is disregarded, but the noise information is kept to update the noise level average. Third, if the noise magnitudes are below the accept thresholds, the frame is kept and passed on for further processing and the noise magnitudes are averaged together with the noise average of the previous frame. This information is used to update thresholds, such that the system adapts to environmental conditions.

When the information about magnitudes of primary tones at $f_1$ and $f_2$, and the noise floor information at and around $f_{dp}$, an online calibration of the level of magnitudes takes place. Several actions occur in the calibration block 114. First, if the noise floor is large when no primary tones are present, the frequency of the primaries is adjusted within predetermined limits. A new $f_{dp}$ is calculated, and the noise content of frequency bins at and around $f_{dp}$ is checked again. This process is repeated until a stable, low noise floor is established. No primary tones are played through the speaker through this process. Once the primaries are presented, they are stepped up to the full output amplitude, as programmed by the user and calibrated in the ear by increasing the output of the codecs 8. No data collection from the ear has taken place yet. At this time, if the level is not reached in a user predetermined time, and at the rate of increase of the primaries, the test is aborted due to lack of fit or the low quality of fit of the probe in the ear canal. Once the proper fit is achieved, and testing begins, data collection takes place. During the entire process of data collection, the levels of tones at $f_1$ and $f_2$ are checked to ensure that they remain within predetermined limits throughout the test. If they exceed those limits, the output is adjusted up or down to compensate until a maximum compensation limit is reached, at which time, the test is aborted and the user is notified. Also, the magnitude at and around $f_{dp}$ is continuously monitored to assure low noise floor, and if necessary, the frequency of the primary tones are adjusted on-line within predetermined limits to avoid the high external noise region. The change in frequencies of the primaries is minimal, and within the specified tolerances of the device 100. and have been shown not to affect the magnitude of the tone within the ear at fdp.

The block 115 is a store/copy buffer. As a frame is collected in new frame buffer 111, a copy of it is saved for processing of the subsequent frames. The buffer 115 receives frame data from new frame buffer 111. The store and copy frame buffer 115 has a variable depth, depending the number of frames averaged together. Buffer 115 provides an output to a block 116 and a block 117. The block 116 operates with the stored previous frames, which are slid by a predetermined amount and the empty spaces padded with zeros for subsequent processing in the averaging old and new frame block 117.

In block 117, the frames are averaged together to reduce the uncorrelated noise present. Theoretically, the noise is reduced by a factor of one over the square root of the number of averaged frames. The frames are averaged in a linear fashion, sample by sample and a new frame is created at the end of the averaging operation. The advantage of this method is that the data is essentially correlated against a slid copy of itself, slid far enough away to avoid averaging the same information content. This provides either a substantial reduction in uncorrelated noise energy for the same amount of sampling time or a substantial reduction in sampling time to obtain the equivalent noise reduction when compared to standard linear averaging.

The minimum limit to the sliding of the data, and to the reuse of old data frame is the autocorrelation function of the data in the frame, which can be predetermined or calculated on-line. This method is equivalent to taking much smaller frames and averaging them together. However, for the purposes of the subsequent Fourier Transformations and filtering taking place, the frame size is required to be large (i.e., 960 samples at 48 kilohertz sampling rate), to obtain several full cycles of each of the tones at f1, f2 and fdp. The problem with taking a large number of very small frames is that the Fourier Transforms or other signal processing methods require several cycles of data for proper operation. The method of the present invention outperforms standard linear averaging of large frames because of the reduction in time as well as providing proper operation of the Fourier Transforms.

The block 118 obtains the averaged data from the block 117, and collects it in a buffer that is used for subsequent processing and signal statistics. The output of the block 118 is digitally filtered in the block 119. The filter 119 removes any remaining high or low frequency components not required for final data presentation.

The averaged and filtered data is converted to frequency domain, in the embodiment illustrated, by using a discrete Fourier Transform in the block 120, and the data then is ready for presentation in block 121. As will be appreciated by those skilled in the art, other signal processing methods are available to convert data, and those other methods are compatible with the device 100.

As indicated above, the device 100 enables the LCD 4 to present information to a user graphically in real time on the device itself, complemented with textual and numeric information about the quality of the fit, amplitudes, frequency, noise floors and other related information.

Operation of the device for ABR testing is shown in Figure 5 and Figure 6. In ABR testing, the magnitude of the fifth peak is the one that is of primary interest, and the device 100 determines the magnitude of the fifth peak by counting zero crossings, after substantial filtering and digital pre-processing. As shown in Figure 5 and Figure 6, the system proceeds to count zero crossings and stores an index of an array element upon determination of a zero crossing. If additional zero crossings are required, the procedure is repeated until the fifth peak is determined. Upon detection, the single waveform is isolated, and the waveform peak is correlated to find the maximum correlation sinusoid. Thereafter, the device 100 determines the time of occurrence of the fifth peak and that value is checked against empirical data to obtain proper correlation.

Numerous variations, within the scope of the appended claims, will be apparent to those skilled in the art in light of the foregoing description and accompanying drawings. For example, the design of the enclosure may vary in other embodiments of the invention. Likewise, LCD display 4 may be replaced with other display devices. As indicated in the specification, we use a discrete Fourier Transform to obtain data for display. Other signal processing methods are compatible with the broader aspects of the invention. These variations are merely illustrative.

**Claims**

1. An auditory screening device, comprising:

   a portable hand-held enclosure;
   a signal processor housed by said enclosure;
   at least one input/output interface housed by said enclosure and operatively coupled to said signal processor;
   a memory module housed by said enclosure, said memory module operatively connected to said signal processor and configured to maintain a plurality of patient records;
   a display screen mounted to said enclosure, said display screen being operatively connected to said signal processor;
   a computer program at least partially contained in said signal processor, said computer program being accessible by a user to perform an otoacoustic emission test and an auditory brainstem response test for a test subject, said memory module maintaining a plurality of test subject records for display on said display screen,

wherein said signal processor is configured to transmit and receive signals through said at least one input/output interface to conduct otoacoustic emission test procedures, and optionally one or more auditory test procedures selected from a group comprising otoreflectance test procedures, auditory brainstem response test procedures, and tympanometry test procedures on a test subject;

**characterised in that** said signal processor is configured to process otoacoustic emission signals received through said input/output interface by performing a time domain sum and average operation using frames and a sliding frame overlap method which comprises reusing data from adjacent frames, which are slid by a predetermined amount, to average with the current frame to reduce uncorrelated noise present in results associated with said otoacoustic emission test procedure.

2. The auditory screening device of Claim 1 wherein said signal processor is further configured to display results associated with a selected test procedure on said display screen.

3. The auditory screening device of Claim 1 wherein said at least one input/output interface is an otoacoustic emission interface, said otoacoustic emission interface including at least one sound transducer configured to present a variety of acoustic signals to a test subject ear, and a microphone configured to receive response acoustic signals from said test subject ear.

4. The auditory screening device of Claim 3 wherein said otoacoustic emission interface is further configured for otoreflectance measurements of a test subject middle ear condition.

5. The auditory screening device of Claim 3 wherein said signal processor is further configured with an OAE simulator program whereby said signal processor is configured to generate simulated $f_{dp}$ tones in response to tones generated by an ear probe.

6. The auditory screening device of Claim 1 wherein said at least one input/output interface is an auditory brainstem interface, said auditory brainstem interface including at least one sound transducer configured to present an auditory stimulus to a test subject ear, and at least one electrode configured to receive response bioelectrical signals from said test subject.

7. The auditory screening device of Claim 1 wherein said at least one input/output interface is a tympanometry interface, said tympanometry interface including at least one electronic control channel, a pump operatively coupled to said electronic control channel for altering a pressure level in a test subject ear, and a pressure sensor configured to measure said pressure level in said test subject ear.

8. The auditory screening device of Claim 1 wherein said signal processor is further configured, for each auditory test procedure, to transmit at least one stimulus signal though said input/output interface.

9. The auditory screening device of Claim 1 further including a display device mounted to said enclosure, said display device being operatively connected to said signal processor, said display device displaying the results of said one or more selected auditory test procedures.

10. The screening device of claim 1 further including a plurality of electrodes for collecting data from a patient, said electrodes being operatively connected to said signal processor.

11. The device of claim 1 wherein said at least one input/output interface is a tympanometry interface operatively connected to said signal processor for recording middle ear pressure on a test subject and adjusting minor middle ear conditions during otoacoustic auditory emission and ABR auditory brainstem response testing.

12. The device of claim 1 wherein said at least one input/output interface is an otoreflectance interface operatively connected to said signal processor for recording and optionally for assessing middle ear conditions on a test subject.

13. The device of claim 3 further including an otoacoustic auditory emission simulator interface operatively connected to said signal processor for testing the integrity of said otoacoustic auditory emission interface.

14. The device of claim 1 further including an infrared interface operatively connected to said signal processor for permitting communication between said signal processor and an external device.

15. The device of claim 9 further including a memory mapped input/output device operatively connected to said memory module and to said signal processor, said display device being operatively connected to said signal processor through said memory mapped device.

16. The device of claim 15 further including a keyboard, said keyboard being operatively connected to said signal processor through said memory mapped device.

17. The device of claim 1 further including a power supply for operating said signal processor, and wherein said power supply is rechargeable.

18. The device of claim 1 wherein an auditory brainstem test signal is determined by digital signal processing and counting zero crossings of correlated internally generated sinusoids.

19. The auditory screening device of claim 1 further comprising:

a computer program at least partial contained within in said signal processor, said computer program being accessible by a user to perform at least an otoacoustic emission test and an auditory brainstem response test for on a test subject.

20. The auditory screening device of claim 19 further including a keyboard for accessing said computer program.

21. The auditory screening device of claim 20 wherein the otoacoustic auditory emission information is recorded by frames, and information from a preceding frame is used in connection with information of a succeeding frame to reduce the signal to noise level in the received signals.

22. The auditory screening device of claim 21 wherein the amount of information employed with a succeeding frame is obtained from the formula:

$$M = \left(\frac{f_n}{f_s - 1}\right) \times \left(\frac{f_s}{f_{dcl} + 1}\right)$$

where $M$ equals overlap number, $f_n$ equals frame number, $f_s$ equals frame size and $f_{dcl}$ equals frame data cycle length.

23. The auditory screening device of claim 22 wherein said computer program further includes tympanometry test procedures conducted independently or in conjunction with otoacoustic auditory emission and auditory brainstem response tests.

24. The auditory screening device of claim 23 wherein the computer program determines data information for the brainstem response test by counting zero crossings of a sinusoid.

25. A method of conducting an OAE test in which a reduced signal to noise ratio is obtained by:

receiving OAE signal information in frames; and
**characterized by**
making a determination to accept a frame, reject data in a frame and update a noise average, or to discard a frame based upon at least one predefined parameter; and
and averaging data in a current accepted frame with data from adjacent frames wherein said data from said at least one adjacent frames is slid by a predetermined amount.

26. The method of claim 25 wherein said step of averaging includes overlapping information from a proceeding frame for use in connection with information from a succeeding frame; and
wherein an overlap amount is determined from the formula:

$$M = \left(\frac{f_n}{f_s - 1}\right) \times \left(\frac{f_s}{f_{dcl} + 1}\right)$$

where $M$ equals overlap number, $f_n$ equals frame number, $f_s$ equals frame size and $f_{dcl}$ equals frame data cycle length.

27. The method of claim 26 further including the step of conducting an auditory brainstem response test for a test subject.

28. The method of claim 27 wherein data for the auditory brainstem response test is obtained by counting zero crossings of an internally generated, correlated sinusoid.

29. The method of claim 25 for conducting an auditory test, further comprising the steps of:

inserting a probe in a patient's ear, said probe including a speaker and a microphone;
connecting said probe to a hand-held device;
generating an auditory signal in said hand-held device;

wherein the step of receiving auditory signal information in frames includes:

• detecting incoming auditory signals generated in said ear via said microphone;
• converting said incoming auditory signals to digital signal data;

• storing said incoming digital signal data in a new frame buffer;

• sizing said new frame buffer to be an integer number of samples of two primary tones at frequencies $f_1$ and $f_2$ and an integer number of samples of a tone produced by said ear at frequency $f_{dp}$;

• passing digital signal data from a single frame to a discrete Fourier transform process to calculate a frequency specific magnitude and phase content of said incoming auditory signal signal;

wherein the step of making a determination includes comparing said calculated magnitude and phase to a table to determine whether to reject the digital signal data, to discard the digital signal data but update a noise table; or to save the digital signal data; and further including the steps of:

collecting said digital signal data until a predetermined number of frames have been saved; converting said averaged data to a frequency domain; and displaying said averaged frequency domain data to the user in a hand-held device in real time.

30. The method of claim 29 further including the step of saving the digital signal data internally in said hand-held device.

31. The method of claim 30 further including the step of sending to the user an indication of the subject passing or failing the test.

32. The method of claim 29 further including the step of transferring said digital signal data from said hand-held device to an external unit.

**Patentansprüche**

1. Hörscreening-Vorrichtung, umfassend:

ein tragbares handgeführtes Gehäuse; einen Signalprozessor, der in dem Gehäuse untergebracht ist; mindestens eine Ein-/Ausgabe-Schnittstelle, die in dem Gehäuse untergebracht ist und betriebsmäßig mit dem Signalprozessor gekoppelt ist; ein Speichermodul, das in dem Gehäuse untergebracht ist, wobei das Speichermodul betriebsmäßig an den Signalprozessor angeschlossen und konfiguriert ist, um eine Vielzahl von Patientenakten zu pflegen; ein Bildschirm, der auf dem Gehäuse installiert ist, wobei der Bildschirm betriebsmäßig an den Signalprozessor angeschlossen ist; ein Computerprogramm, das mindestens teilweise in dem Signalprozessor enthalten ist, wobei das Computerprogramm für einen Benutzer zugänglich ist, um einen otoakustischen Emissionstest und einen Hirnstammaudiometrietest für eine Testperson auszuführen, wobei das Speichermodul eine Vielzahl von Testpersonakten zum Anzeigen auf dem Bildschirm pflegt,

wobei der Signalprozessor konfiguriert ist, um Signale über die mindestens eine Ein-/Ausgabe-Schnittstelle zu senden und zu empfangen, um otoakustische Emissionsprüfverfahren und wahlweise eine oder mehrere Gehörprüfverfahren, die aus einer Gruppe gewählt werden, welche akustische Reflektanz-Prüfverfahren, Hirnstammaudiometrie-Prüfverfahren und Tympanometrie-Prüfverfahren umfasst, bei einer Testperson durchzuführen; **dadurch gekennzeichnet, dass** der Signalprozessor konfiguriert ist, um otoakustische Emissionssignale zu verarbeiten, die über die Ein-/Ausgabe-Schnittstelle empfangen werden, indem er eine Zeitbereichsumme und eine Mittelwertbildung ausführt unter Verwendung von Rahmen und eines gleitenden Rahmenüberschneidungsverfahrens, das die Wiederverwendung von Daten umfasst, die aus angrenzenden Rahmen stammen, die um einen vorherbestimmten Betrag verschoben werden, um sie mit dem derzeitigen Rahmen zu mitteln, um das unkorrelierte Rauschen zu reduzieren, das in den Ergebnissen vorkommt, die mit dem otoakustischen Emissionsprüfverfahren verbunden sind.

2. Hörscreening-Vorrichtung nach Anspruch 1, wobei der Signalprozessor ferner konfiguriert ist, um die mit einem ausgewählten Prüfverfahren verbundenen Ergebnisse auf dem Bildschirm anzuzeigen.

3. Hörscreening-Vorrichtung nach Anspruch 1, wobei die mindestens eine Ein-/Ausgabe-Schnittstelle eine otoakustische Emissionsschnittstelle ist, wobei die otoakustische Emissionsschnittstelle mindestens einen Schallwandler, der konfiguriert ist, um dem Ohr einer Testperson diverse akustische Signale zu präsentieren, und ein Mikrofon, das konfiguriert ist, um akustische Antwortsignale aus dem Ohr der Testperson zu empfangen, umfasst.

4. Hörscreening-Vorrichtung nach Anspruch 3, wobei die otoakustische Emissionsschnittstelle ferner für akustische Reflektanzmessungen einer Mittelohrerkrankung einer Testperson konfiguriert ist.

5. Hörscreening-Vorrichtung nach Anspruch 3, wobei der Signalprozessor ferner mit einem OAE-Simulationsprogramm konfiguriert ist, so dass der Signalprozessor konfiguriert ist, um simulierte Töne $f_{dp}$ als Antwort auf von einer Ohrsonde erzeugte Töne zu erzeugen.

6. Hörscreening-Vorrichtung nach Anspruch 1, wobei die mindestens eine Ein-/Ausgabe-Schnittstelle eine Schnittstelle zum Gehörgehirnstamm ist, wobei die Schnittstelle zum Gehörgehirnstamm mindestens einen Schallwandler, der konfiguriert ist, um einen akustischen Reiz für das Ohr einer Testperson zu präsentieren, und mindestens eine Elektrode, die konfiguriert ist, um bioelektrische Antwortsignale von der Testperson zu empfangen, umfasst.

7. Hörscreening-Vorrichtung nach Anspruch 1, wobei die mindestens eine Ein-/Ausgabe-Schnittstelle eine Tympanometrie-Schnittstelle ist, wobei die Tympanometrie-Schnittstelle mindestens einen elektronischen Steuerkanal, eine Pumpe, die betriebsmäßig mit dem elektronischen Steuerkanal gekoppelt ist, um einen Druckpegel im Ohr einer Testperson zu ändern, und einen Drucksensor, der konfiguriert ist, um den Druckpegel im Ohr der Testperson zu messen, umfasst.

8. Hörscreening-Vorrichtung nach Anspruch 1, wobei der Signalprozessor ferner konfiguriert ist, um für jedes Gehörprüfverfahren mindestens ein Reizsignal durch die Ein-/Ausgabe-Schnittstelle zu übertragen.

9. Hörscreening-Vorrichtung nach Anspruch 1, ferner umfassend eine Anzeigevorrichtung, die auf dem Gehäuse angebracht ist, wobei die Anzeigevorrichtung betriebsmäßig an den Signalprozessor angeschlossen ist, wobei die Anzeigevorrichtung die Ergebnisse des einen oder der mehreren ausgewählten Gehörprüfverfahren anzeigt.

10. Screening-Vorrichtung nach Anspruch 1, ferner umfassend eine Vielzahl von Elektroden, zum Sammeln von Daten bei einem Patienten, wobei die Elektroden betriebsmäßig an den Signalprozessor angeschlossen sind.

11. Vorrichtung nach Anspruch 1, wobei die mindestens eine Ein-/Ausgabe-Schnittstelle eine Tympanometrie-Schnittstelle ist, die betriebsmäßig an den Signalprozessor angeschlossen ist, um einen Mittelohrdruck bei einer Testperson zu messen und leichte Mittelohrerkrankungen während der otoakustischen Emission und den Hirnstammaudiometrietests ABR zu regulieren.

12. Vorrichtung nach Anspruch 1, wobei die mindestens eine Ein-/Ausgabe-Schnittstelle eine akustische Reflektanzschnittstelle ist, die betriebsmäßig an den Signalprozessor angeschlossen ist, um die Mittelohrerkrankungen bei einer Testperson aufzuzeichnen und wahlweise zu beurteilen.

13. Vorrichtung nach Anspruch 3, ferner umfassend eine Schnittstelle eines otoakustischen Emissionssimulators, die betriebsmäßig an den Signalprozessor angeschlossen ist, um die Integrität der otoakustischen Emissionsschnittstelle zu testen.

14. Vorrichtung nach Anspruch 1, ferner umfassend eine Infrarotschnittstelle, die betriebsmäßig an den Signalprozessor angeschlossen ist, um eine Verbindung zwischen dem Signalprozessor und einer externen Vorrichtung zu ermöglichen.

15. Verrichtung nach Anspruch 9, ferner umfassend eine speicheradressierte Ein-/Ausgabe-Vorrichtung, die betriebsmäßig an das Speichermodul und an den Signalprozessor angeschlossen ist, wobei die Anzeigevorrichtung betriebsmäßig an den Signalprozessor über die speicheradressierte Vorrichtung angeschlossen ist.

16. Vorrichtung nach Anspruch 15, ferner umfassend eine Tastatur, wobei die Tastatur betriebsmäßig an den Signalprozessor über die speicheradressierte Vorrichtung angeschlossen ist.

17. Vorrichtung nach Anspruch 1, ferner umfassend eine Energieversorgung, um den Signalprozessor zu betreiben, und wobei die Energieversorgung aufladbar ist.

18. Vorrichtung nach Anspruch 1, wobei ein Hirnstammaudiometrie-Testsignal durch eine digitale Signalverarbeitung und das Zählen der Nulldurchgänge von korrelierten, intern erzeugten Sinuswellen bestimmt wird.

19. Hörscreening-Vorrichtung nach Anspruch 1, ferner umfassend:

ein Computerprogramm, das mindestens teilweise in dem Signalprozessor enthalten ist, wobei das Computerprogramm für einen Benutzer zugänglich ist, um mindestens einen otoakustischen Emissionstest und einen Hirnstammaudiometrietest für eine Testperson auszuführen.

20. Hörscreening-Vorrichtung nach Anspruch 19, ferner umfassend eine Tastatur, um auf das Computerprogramm zuzugreifen.

21. Herscreening-Vorrichtung nach Anspruch 20, wobei die otoakustischen Emissionsinformationen in Rahmen aufgezeichnet werden, und Informationen aus einem vorhergehenden Rahmen zusammen mit Informationen eines nachfolgenden Rahmen verwendet werden, um das Signal/Rausch-Verhältnis in den empfangenen Signalen zu reduzieren.

**22.** Hörscreening-Vorrichtung nach Anspruch 21, wobei die Menge der Informationen, die mit einem nachfolgenden Rahmen verwendet werden, durch folgende Formel erhalten wird:

$$M = \left(\frac{f_n}{f_s - 1}\right) \times \left(\frac{f_s}{f_{dcl} + 1}\right)$$

wobei M der Überschneidungswert, $f_n$ die Rahmenzahl, $f_s$ die Rahmengröße und $f_{dcl}$ die Datenzykluslänge des Rahmens ist.

**23.** Hörscreening-Vorrichtung nach Anspruch 22, wobei das Computerprogramm ferner Tympanometrie-Prüfverfahren umfasst, die unabhängig oder zusammen mit otoakustischen Emissionstests und Hirnstammaudiometrietests ausgeführt wird.

**24.** Hörscreening-Vorrichtung nach Anspruch 23, wobei das Computerprogramm Dateninformationen für den Hirnstammaudiometrietest bestimmt, indem es die Nulldurchgänge einer Sinuswelle zählt.

**25.** Verfahren zum Durchführen eines OAE-Tests, wobei ein reduziertes Signal/Rausch-Verhältnis durch folgenden Schritt erzielt wird:

Empfangen von OAE-Signalinformationen in Rahmen; und

**gekennzeichnet durch** folgende Schritte:

Vornehmen einer Bestimmung, um einen Rahmen anzunehmen, Daten in einem Rahmen zurückzuweisen und einen Rauschmittelwert zu aktualisieren, oder um einen Rahmen auf der Basis mindestens eines vorgegebenen Parameters zu verwerfen; und
Mitteln von Daten in einem derzeit angenommenen Rahmen mit Daten aus angrenzenden Rahmen, wobei die Daten aus dem mindestens einen angrenzenden Rahmen um einen vorherbestimmten Betrag verschoben sind.

**26.** Verfahren nach Anspruch 25, wobei der Schritt der Mittelwertbildung das Überschneiden von Informationen aus einem vorhergehenden Rahmen für eine Verwendung in Zusammenhang mit Informationen aus einem nachfolgenden Rahmen umfasst; und wobei ein Überschneidungsbetrag aus folgender Formel bestimmt wird:

$$M = \left(\frac{f_n}{f_s - 1}\right) \times \left(\frac{f_s}{f_{dcl} + 1}\right)$$

wobei M der Überschneidungswert, $f_n$ die Rahmenzahl, $f_s$ die Rahmengröße und $f_{dcl}$ die Datenzykluslänge des Rahmens ist.

**27.** Verfahren nach Anspruch 26, ferner umfassend den Schritt des Durchführens eines Hirnstammaudiometrietests für eine Testperson.

**28.** Verfahren nach Anspruch 27, wobei die Daten für den Hirnstammaudiometrietest erzielt werden, indem die Nulldurchgänge einer intern erzeugten, korrelierten Sinuswelle gezählt werden.

**29.** Verfahren nach Anspruch 25 zum Durchführen eines Gehörtests, ferner umfassend folgende Schritte:

Einsetzen einer Sonde in das Ohr eines Patienten, wobei die Sonde einen Lautsprecher und ein Mikrofon umfasst;
Anschließen der Sonde an eine handgeführte Vorrichtung;
Erzeugen eines akustischen Signals in der handgeführten Vorrichtung;

wobei der Schritt des Empfangens der akustischen Signalinformationen in Rahmen folgende Schritte umfasst:

- Erfassen über das Mikrofon von ankommenden akustischen Signalen, die in dem Ohr erzeugt werden;
- Umwandeln der ankommenden akustischen Signale in digitale Signaldaten;
- Speichern der ankommenden digitalen Signaldaten in einem neuen Rahmenpuffer;
- Bemessen des neuen Rahmenpuffers, damit er einer Ganzzahl von Proben von zwei Primärtönen auf den Frequenzen $f_1$ und $f_2$ und einer Ganzzahl von Proben eines Tons, der von dem Ohr auf einer Frequenz $f_{dp}$ erzeugt wird, entspricht;
- Weitergeben der digitalen Signaldaten eines einzelnen Rahmens an einen diskreten Fourier-Transformationsvorgang, um eine frequenzspezifische Größe und einen Phaseninhalt des ankommenden akustischen Signals zu berechnen;

wobei der Schritt des Vornehmens einer Bestimmung das Vergleichen der berechneten Größe und Phase mit einer Tabelle umfasst, um zu bestimmen, ob die digitalen Signaldaten zurückzuweisen sind, ob die digitalen Signaldaten zu verwerfen sind, aber eine Rauschtabelle zu aktualisieren ist; oder ob die digitalen Signaldaten zu speichern sind; und ferner umfassend folgende Schritte:

Sammeln der digitalen Signaldaten, bis eine vorherbestimmte Anzahl von Rahmen gespeichert ist;

Umwandeln der gemittelten Daten in einen Frequenzbereich; und

Anzeigen der gemittelten Frequenzbereichdaten für den Benutzer in einer handgeführten Vorrichtung in Echtzeit.

**30.** Verfahren nach Anspruch 29, ferner umfassend den Schritt des Speicherns der digitalen Signaldaten innerhalb der handgeführten Vorrichtung.

**31.** Verfahren nach Anspruch 30, ferner umfassend den Schritt des Sendens an den Benutzer einer Angabe, ob die Testperson den Test bestanden hat oder nicht.

**32.** Verfahren nach Anspruch 29, ferner umfassend den Schritt des Übertragens der digitalen Signaldaten von der handgeführten Vorrichtung an eine externe Einheit.

## Revendications

**1.** Dispositif de dépistage auditif, comprenant :

un boîtier tenu à la main portable ;

un processeur de signaux logé par ledit boîtier ;

au moins une interface d'entrée/sortie logée par ledit boîtier et couplée de façon opérationnelle audit processeur de signaux ;

un module de mémoire logé par ledit boîtier, ledit module de mémoire étant connecté de façon opérationnelle audit processeur de signaux et configuré pour entretenir une pluralité de dossiers de patient ;

un écran d'affichage installé sur ledit boîtier, ledit écran d'affichage étant connecté de façon opérationnelle audit processeur de signaux ;

un programme informatique contenu au moins en partie dans ledit processeur de signaux, ledit programme informatique étant accessible par un utilisateur pour effectuer un test d'émission otoacoustique et un test de réponse auditive du tronc cérébral pour une personne testée, ledit module de mémoire entretenant une pluralité de dossiers de personnes testées pour un affichage sur ledit écran d'affichage,

dans lequel ledit processeur de signaux est configuré pour émettre et recevoir des signaux à travers ladite au moins une interface d'entrée/sortie pour réaliser des procédures de test d'émission otoacoustique, et en option une ou plusieurs procédures de test auditif sélectionnées dans un groupe comprenant des procédures de test d'otoréflectance, des procédures de test de réponse auditive du tronc cérébral et des procédures de test de tympanométrie, chez une personne testée ;

**caractérisé en ce que** ledit processeur de signaux est configuré pour traiter des signaux d'émission otoacoustique reçus à travers ladite interface d'entrée/ sortie en effectuant une somme de domaine temporel et une opération de calcul de moyenne en utilisant des trames et un procédé de recouvrement de trames mobiles qui comprend la réutilisation des données provenant de trames adjacentes qui sont déplacées d'une quantité prédéterminée, pour faire la moyenne avec la trame actuelle afin de réduire un bruit non corrélé présent dans les résultats associés à ladite procédure de test d'émission otoacoustique.

**2.** Dispositif de dépistage auditif selon la revendication 1, dans lequel ledit processeur de signaux est configuré pour afficher des résultats associés à une procédure de test sélectionnée sur ledit écran d'affichage.

**3.** Dispositif de dépistage auditif selon la revendication 1, dans lequel ladite au moins une interface d'entrée/ sortie est une interface d'émission otoacoustique, ladite interface d'émission otoacoustique comprenant au moins un transducteur sonore configuré pour présenter divers signaux acoustiques à l'oreille d'une personne testée, et un microphone configuré pour recevoir des signaux acoustiques de réponse en provenance de ladite oreille d'une personne testée.

**4.** Dispositif de dépistage auditif selon la revendication 3, dans lequel ladite interface d'émission otoacoustique est en outre configurée pour des mesures d'otoréflectance d'une pathologie de l'oreille moyenne d'une personne testée.

**5.** Dispositif de dépistage auditif selon la revendication 3, dans lequel ledit processeur de signaux est en outre configuré avec un programme de simulation OAE, de manière à ce que ledit processeur de signaux soit configuré pour générer des sons $f_{dp}$ simulés en réponse à des sons générés par une sonde auriculaire.

**6.** Dispositif de dépistage auditif selon la revendication 1, dans lequel ladite au moins une interface d'entrée/ sortie est une interface de tronc cérébral auditif, ladite interface de tronc cérébral auditif comprenant au moins un transducteur sonore configuré pour présenter un stimulus auditif à l'oreille d'une personne testée, et au moins une électrode configurée pour recevoir des signaux bioélectriques de réponse de la part de ladite personne testée.

**7.** Dispositif de dépistage auditif selon la revendication 1, dans lequel ladite au moins une interface d'entrée/sortie est une interface de tympanométrie, ladite interface de tympanométrie comprenant au moins un canal de commande électronique, une pompe couplée de façon opérationnelle audit canal de commande électronique pour modifier un niveau de pression dans l'oreille d'une personne testée, et un capteur de pression configuré pour mesurer ledit niveau de pression dans l'oreille de ladite personne testée.

**8.** Dispositif de dépistage auditif selon la revendication 1, dans lequel ledit processeur de signaux est en outre configuré pour émettre pour chaque procédure de test auditif au moins un signal de stimulation à travers ladite interface d'entrée/sortie.

**9.** Dispositif de dépistage auditif selon la revendication 1, comprenant en outre un dispositif d'affichage monté sur ladite enceinte, ledit dispositif d'affichage étant connecté de façon opérationnelle audit processeur de signaux, ledit dispositif d'affichage affichant les résultats de ladite une ou desdites plusieurs procédures de test auditif.

**10.** Dispositif de dépistage selon la revendication 1, comprenant en outre une pluralité d'électrodes pour collecter des données auprès d'un patient, lesdites électrodes étant connectées de façon opérationnelle audit processeur de signaux.

**11.** Dispositif selon la revendication 1, dans lequel ladite au moins une interface d'entrée/sortie est une interface de tympanométrie connectée de façon opérationnelle audit processeur de signaux pour enregistrer une pression de l'oreille moyenne chez une personne testée et réguler des pathologies mineures de l'oreille moyenne pendant l'émission auditive otoacoustique et les tests de réponse auditive du tronc cérébral ABR.

**12.** Dispositif selon la revendication 1, dans lequel ladite au moins une interface d'entrée/sortie est une interface d'otoréflectance connectée de façon opérationnelle audit processeur de signaux pour enregistrer et en option évaluer des pathologies de l'oreille moyenne chez une personne testée.

**13.** Dispositif selon la revendication 3, comprenant en outre une interface de simulateur d'émission auditive otoacoustique, connectée de façon opérationnelle audit processeur de signaux pour tester l'intégrité de ladite interface d'émission auditive otoacoustique.

**14.** Dispositif selon la revendication 1, comprenant en outre une interface infrarouge connectée de façon opérationnelle audit processeur de signaux pour permettre une communication entre ledit processeur de signaux et un dispositif externe.

**15.** Dispositif selon la revendication 9, comprenant en outre un dispositif d'entrée/sortie mappé en mémoire, connecté de façon opérationnelle audit module de mémoire et audit processeur de signaux, ledit dispositif d'affichage étant connecté de façon opérationnelle audit processeur de signaux à travers ledit dispositif mappé en mémoire.

**16.** Dispositif selon la revendication 15, comprenant en outre un clavier, ledit clavier étant connecté de façon opérationnelle audit processeur de signaux à travers ledit dispositif mappé en mémoire.

**17.** Dispositif selon la revendication 1, comprenant en outre une alimentation électrique pour exploiter ledit processeur de signaux, et dans lequel ladite alimentation électrique est rechargeable.

**18.** Dispositif selon la revendication 1, dans lequel un signal de test de tronc cérébral auditif est déterminé par un traitement de signaux numériques et un comptage des passages par zéro d'ondes sinusoïdales corrélées, générées en interne.

**19.** Dispositif de dépistage auditif selon la revendication 1, comprenant en outre :

un programme informatique contenu au moins en partie dans ledit processeur de signaux, ledit programme informatique étant accessible par un utilisateur pour effectuer au moins un test d'émission otoacoustique et un test de réponse auditive du tronc cérébral pour une personne testée.

**20.** Dispositif de dépistage auditif selon la revendication 19, comprenant en outre un clavier pour accéder audit programme informatique.

**21.** Dispositif de dépistage auditif selon la revendication 20, dans lequel les informations d'émission auditive otoacoustique sont enregistrées par trames, et des informations provenant d'une trame précédente sont utilisées en relation avec des informations d'une trame suivante pour réduire le rapport signal/bruit dans les signaux reçus.

**22.** Dispositif de dépistage auditif selon la revendication 21, dans lequel la quantité d'informations utilisées avec une trame suivante est obtenue à partir de la formule :

$$M = \left( \frac{f_n}{f_a - 1} \right) \times \left( \frac{f_s}{f_{dc1} + 1} \right)$$

où M est égal à la valeur de recouvrement, $f_n$ est

égal au numéro de trame, $f_s$ est égal à la taille de la trame et $f_{dcl}$ est égal à la longueur de cycle de données de la trame.

23. Dispositif de dépistage auditif selon la revendication 22, dans lequel ledit programme informatique comprend en outre des procédures de test de tympanométrie réalisées indépendamment ou en conjonction avec une émission auditive otoacoustique et des tests de réponse auditive du tronc cérébral.

24. Dispositif de dépistage auditif selon la revendication 23, dans lequel le programme informatique détermine des informations de données pour le test de réponse du tronc cérébral en comptant les passages par zéro d'une onde sinusoïdale.

25. Procédé de réalisation d'un test OAE, dans lequel un rapport signal/bruit réduit est obtenu par l'étape consistant à :

recevoir des informations de signal OAE dans des trames ; et

**caractérisé par** les étapes consistant à :

réaliser une détermination pour accepter une trame, rejeter des données dans une trame et mettre à jour une moyenne de bruit, ou abandonner une trame sur la base d'au moins un paramètre prédéfini ; et
établir la moyenne des données dans une trame acceptée actuelle avec des données provenant de trames adjacentes, dans lequel lesdites données provenant de ladite au moins une trame adjacente sont décalées d'une quantité prédéterminée.

26. Procédé selon la revendication 25, dans lequel ladite étape d'établissement de la moyenne comprend le recouvrement des informations provenant d'une trame précédente pour une utilisation en relation avec des informations provenant d'une trame suivante ; et dans lequel une quantité de recouvrement est déterminée à partir de la formule :

$$M = \left(\frac{f_n}{f_s - 1}\right) \times \left(\frac{f_s}{f_{dcl} + 1}\right)$$

où M est égal à la valeur de recouvrement, $f_n$ est égal au numéro de trame, $f_s$ est égal à la taille de la trame et $f_{del}$ est égal à la longueur de cycle de données de la trame.

27. Procédé selon la revendication 26, comprenant en outre l'étape consistant à réaliser un test de réponse auditive du tronc cérébral pour une personne testée.

28. Procédé selon la revendication 27, dans lequel les données pour le test de réponse auditive du tronc cérébral sont obtenues en comptant les passages par zéro d'une onde sinusoïdale corrélée, générée en interne.

29. Procédé selon la revendication 25, pour réaliser un test auditif, comprenant en outre les étapes consistant à :

insérer une sonde dans l'oreille d'un patient, ladite sonde comprenant un haut-parleur et un microphone ;
connecter ladite sonde à un dispositif tenu à la main ;
générer un signal auditif dans ledit dispositif tenu à la main ;

dans lequel l'étape consistant à recevoir des informations de signal auditif dans des trames comprend les étapes consistant à :

- détecter des signaux auditifs entrants générés dans ladite oreille par l'intermédiaire dudit microphone ;
- convertir lesdits signaux auditifs entrants en données de signaux numériques ;
- stocker lesdites données de signaux numériques entrants dans un nouveau tampon de trame ;
- dimensionner ledit nouveau tampon de trame pour correspondre à un nombre entier d'échantillons de deux sons primaires à des fréquences $f_1$ et $f_2$ et à un nombre entier d'échantillons d'un son produit par ladite oreille à une fréquence $f_{dp}$ ;
- faire passer des données de signaux numériques provenant d'une trame unique à un processus de transformée de Fourier discrète pour calculer une grandeur spécifique à la fréquence et un contenu de phase dudit signal auditif entrant ;

dans lequel l'étape consistant à réaliser une détermination comprend la comparaison de ladite grandeur calculée et de ladite phase à un tableau pour déterminer s'il convient de rejeter les données de signaux numériques, d'écarter les données de signaux numériques mais de mettre à jour une table de bruit ; ou de sauvegarder les données de signaux numériques ; et
comprenant en outre les étapes consistant à :

collecteur lesdites données de signaux numériques jusqu'à à ce qu'un nombre de trames prédéterminées ait été sauvegardé ;

convertir lesdites données moyennées en un domaine fréquentiel ; et

afficher lesdites données de domaine fréquentiel moyennées à l'attention de l'utilisateur dans un dispositif tenu à la main, en temps réel.

**30.** Procédé selon la revendication 29, comprenant en outre l'étape consistant à sauvegarder les données de signaux numériques en interne dans ledit dispositif tenu à la main.

**31.** Procédé selon la revendication 30, comprenant en outre l'étape consistant à envoyer à l'utilisateur une indication pour savoir si la personne testée passe le test ou non.

**32.** Procédé selon la revendication 29, comprenant en outre l'étape consistant à transférer lesdites données de signaux numériques dudit dispositif tenu à la main à une unité externe.

FIG.1

FIG.2

FIG.3

FIG.4

EP 1 187 550 B1

```
┌─────────────────────┐
│  FIND AND SUBTRACT  │
│         DC          │
│   OFFSET (MEAN)     │
└─────────────────────┘
```

LOCATE ZERO CROSSINGS
ODD ZERO CROSSING = POSITIVE SLOPE
EVEN ZERO CROSSING = NEGATIVE SLOPE

```
┌─────────────────────┐
│  MULTIPLY PREVIOUS  │
│    ARRAY ELEMENT*   │
│  CURRENT ARRAY ELEMENT │
└─────────────────────┘
```

YES?          <0?          NO? ──────►

```
┌─────────────────────────────┐
│     CHECK IF NEXT (50)       │
│   ELEMENTS CHANGE SIGN       │
│           -OR-               │
│    PREVIOUS 50 ELEMENTS      │
│  CHANGE SIGN (IF 50 EXIST)   │
└─────────────────────────────┘
```

YES?                   └──►NO? NOT AN ACCEPTABLE
                              ZERO CROSSING

```
┌─────────────────────┐
│ A ZERO CROSSING HAS │
│    BEEN LOCATED.    │
│    STORE INDEX OF   │
│    ARRAY ELEMENT    │
└─────────────────────┘
```

```
┌─────────────────────┐
│ CHECK IF MORE ZEROS │      ──► YES?
│ NEED TO BE REQUIRED │
└─────────────────────┘
```

NO?

```
┌──────────────────────────────┐
│   NO? PROCEED TO SINUSOID     │
│ CORRELATION AND PEAK LOCATION │
└──────────────────────────────┘
```

# FIG.5

SINUSOID CORRELATION AND PEAK DETECTION

```
┌─────────────────────────┐
│  ISOLATE SINGLE WAVEFORM │
│       PEAK BETWEEN       │
│    CONSECUTIVE ZEROS     │
└─────────────────────────┘
             │
             ▼
┌─────────────────────────────┐
│  GENERATE (100) HALF-SINUSOIDS. │
│ EACH SINUSOID VARIES IN FREQUENCY │
│   BY (0.000025) CYCLES/SAMPLE   │
└─────────────────────────────┘
             │
             ▼
┌─────────────────────────────┐
│  CORRELATE WAVEFORM PEAK WITH  │
│ EACH SINUSOID AND FIND MAXIMUM │
│     CORRELATION SINUSOID.      │
└─────────────────────────────┘
             │
             ▼
┌─────────────────────────────┐
│     LOCATE TIME OF PEAK BY      │
│      MULTIPLYING 1/SAMPLING     │
│ FREQUENCY TIMES INDEX OF LEFT SIDE │
│ ZERO OF INITIAL WAVEFORM PLUS THE │
│   LENGTH OF ONE QUARTER OF THE  │
│     SINUSOID WITH MAXIMUM.      │
└─────────────────────────────┘
```

FIG.6

N FRAMES

FRAME SIZE
=960 FOR DFT

$F_{DATA-EQUAL-PHASE-BOUNDARY}$

$F_{DATA}$ CYCLE LENGTH

FIG.7

EP 1 187 550 B1

FILTER

D/A

D/A

EAR PROBE

OUT

OUT

MIC

A/D

NEW FRAME BUFFER

POINT DFT

NOISE CALCULATION

CALIBRATION CALCULATION

STORE COPY OF OLD FRAME > OLD

SLIDE OLD FRAME

AVERAGE NEW AND OLD FRAME OVERLAP AVE

FINAL AVERAGE BUFFER

DIGITAL FILTER

DFT DSP

GRAPHICAL REP. OF NOISE, $f_1$, $f_2$ AND $f_{dp}$ ON LCD SCREEN

FIG.8

**EP 1 187 550 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5601091 A
- US 5916174 A
- US 5885225 A